# EUROPEAN PATENT SPECIFICATION

(11) **EP 2 968 822 B1**
(45) Date of publication and mention of the grant of the patent: **09.05.2018**
(21) Application number: 14767532.6
(22) Date of filing: 13.03.2014
(51) Int. Cl.: A61M 16/06

(54) **METHOD OF MAKING A FACIAL MASK APPARATUS**
VERFAHREN ZUR HERSTELLUNG EINER GESICHTSMASKENVORRICHTUNG
PROCÉDÉ DE FABRICATION D'UN APPAREIL DE MASQUE FACIAL

(30) Priority: 15.03.2013 US 201313835059
(43) Date of publication of application: 20.01.2016
(73) Proprietor: Davis, Lucy Carol, Chapel Hill, NC 27514 (US)
(72) Inventor: Davis, Lucy Carol, Chapel Hill, NC 27514 (US)
(74) Representative: Murgitroyd & Company
(86) International application number: PCT/US2014/025473
(87) International publication number: WO 2014/151324

(56) References cited:
- WO-A1-00/35525
- WO-A1-2009/063402
- WO-A1-2011/049548
- WO-A1-2013/026091
- US-A- 5 492 116
- US-A1- 2006 023 228
- US-A1- 2006 058 632
- US-A1- 2009 266 362
- US-A1- 2010 199 992
- US-A1- 2012 305 003
- US-B1- 6 543 450

## Description

### BACKGROUND OF THE INVENTION

### 1. Field of the Invention

The present invention relates to methods of making a face mask apparatus, and more particularly, a face mask apparatus for sleep apnea treatment.

### 2. Description of the Prior Art

It is known in the prior art to provide sleep apnea treatment medical devices. However, they are typically not customized and create impressions and indentations on the face of the user during and/or after use. Currently, masks are made from plastic, and are adjusted by either foam or gel to suit the comfort level of the patient. In addition, the gel or foam is also utilized to form the seal between the mask and the patient's skin. For reasons discussed above, the seal is an important part of the effectiveness of the mask.

It is also known in the art to provide customizable masks for facial application. It is further known in the art to use computer aided design for custom face mask design and manufacture. And it is also known to provide three-dimensional (3D) facial data for use for fabrication of a custom fit mask for medical procedures.

Examples of relevant prior art reference documents include the following:
U.S. Pat. Pub. No. 2012/0305003 for "Rapid production of customized masks" by inventor Phillip E. Mark, filed October 21, 2009, describes a system designed for the rapid preparation of anatomically customized mask employing data from a patient. The data may take the form of a multidimensional image of a target area of a patient's face obtained by optical 3 dimensional imaging, or a dot or line scan form laser imaging, pattern laser photography or stereo photography. Also disclosed is a mask that is made of a thin layer, so it is lightweight and closely hugs the targeted region upon which it rests (e.g. the nasal region).

U.S. Pat. No. 5,280,305 for "Method and apparatus for forming a stylized, three-dimensional object" by inventors Monroe, *et al*., filed October 30, 1992, describes an electronic device capable of creating a masquerade type mask utilizing a printer and a heating system. Includes a printing system that uses an electronic signal representing the mask and printing the image. The printing system reads an external signal from an imaging device to generate a three-dimensional object. The material used is a heat settable material, so the material may be manipulated by heat to form the shape desired for the mask. The printer prints the desired image onto the 3D mask.

U.S. Pat. No. 4,985,116 for "Three dimensional plating or etching process and masks therefor" by inventors Mettler, *et al*., filed February 23, 1990, describes a method for fabricating a mask that utilizes a transparent vacuum formable material, adding ink and then removing the ink after vacuuming to reveal transparent areas of the mask: The ink is removed by a laser. The mask is an "insulative" substrate material molded to a predetermined shape: A flexible material is added to the "insulative" substrate mold. A vacuum is applied between the "insulative" substrate material and the flexible material to create the fabricated mask. Etching on the plating substrate area and then removing the plated areas to expose the plated substrate.

U.S. Pat. No. 8,020,276 for "System and method for custom-orienting a medical mask to an oral appliance" by inventor W. Keith Thornton, filed November 29, 2007, describes a medical mask utilized to cover a patient's nose and mouth. The mask is configured to prevent gas from escaping and is configured to cover the nose and mouth. The invention further includes transforming a non-deformable material into deformable material. The deformable material is a thermoplastic polymer. The thermoplastic polymer is a polycaprolactone polymer. The medical mask covers the patient's nose and mouth. The orientation of the mask is customized to cover the stated portion of the face while also orienting it to attach to an oral appliance. The medical mask has a sealing portion configured to the contours of the face to prevent gas from leaking out of the mask.

U.S. Pat. No. 8,254,637 for "Mask fitting system and method" by inventors Abourizk, *et al*., filed July 26, 2007, describes a system and methods for selecting a mask system for a patient, where certain embodiments include generating 3D contours of patients and selecting mask systems based at least on those contours. The patent further describes a mask fitting system comprising: an image and/or video acquiring device to capture plural images of at least a portion of the patient's face; a processor operable to process the images to determine a physical characteristic of the patient's face; a display to show the images of the selection mask systems; and a head support and camera mount assembly; a base; an upright provided to the base, the upright including a chin support adapted to support the patient's chin in use; an arm extending outwardly from the upright to support the image acquiring device; and servo motors to automate movement of the arm. Additionally claims a method of selecting, from a plurality of selection mask systems, at least two mask systems for a patient.

U.S. Pat. No. 7,827,038 for "Mask fitting system and method" by inventors Richard *et al*., filed June 6, 2005, describes a mask fitting system, which bases the mask on the scanned in data received from the patient. The data is scanned in using a handheld scanner or a 3D scanner to create the mask. The best fit mask for the patient might include multiple mask recommendations. The mask fitting system also includes one terminal to receive patient specific data; the data includes at least one two dimensional profile image and at least one two dimensional front image of the patient; the patient specific data includes the depth, length, and width of the patient's nose. One terminal including a 3D mask fitting system to represent the facial features of the patient. The computer reads the data generated from the 3D mask fitting system and communicates the data to the mask database. A contact cushion contouring device is used to further provide the 3-D representation of the patient's face. The data recorded is compared to the patient's facial dimensions and the mask system provides a best fit mask. The mask fitting system provides the facial dimensions from the scanner and produces a best fit mask for the patient.

U.S. Pat. Pub. No. 2006/0023228 for "Custom fit facial, nasal, and nostril masks" by inventor Zheng Jason Geng, filed June 10, 2005, describes generating 3D facial data to fabricate a custom fit mask for medical procedures, the mask including: Fabricating a facial mask custom fit for a patient. The mask covers the patient's nostril or a facial mask. The mask is generated utilizing a 3D imaging system. The 3D imaging system utilizes the Kanade Lucas Tomasi extraction algorithm. The algorithm defines the portion of the face and generates a 3D facial data set to create a custom fit facial mask for the desired medical procedure. The medical procedure involves expelling gas or air. The mask is a facial mask or covers the patient's nostrils. There is a support mechanism for attaching the facial mask to the patient's head.

U.S. Pat. Pub. No. 2004/0263863 for "System and method for design and manufacture of custom face masks" by inventors Rogers, *et al*., filed January 27, 2004, describes a method for forming a face mask utilizing computer aided design. The custom mask is designed based on facial topography and the topography information is passed to a manufacturing device. Further teaches a scanning device, utilizing a laser and a camera to scan the head. Attached is at least one position sensor to keep the head from moving. The scanned information is fed to a computer where the computer determines the topography of the client's face. The construction of the mask includes: A solid model of the face, to which an intermediate layer is applied. The face mask is formed by applying mask forming material to the intermediate layer. The face mask is then separated from the solid model of the face.

WO 00/35525 for "Respiratory mask" by Collin Stuart Anderson, filed December 8, 1999 and published June 22, 2000, describes a respiratory mask comprising areas of semi-ridged and semi-soft silicone moulded from an exact impression of the user's face, covering both cheeks, forehead, and nose including bridge, comprising a gas intake box, incorporating nostril extensions, located beneath the nose, reducing the dead air space enclosed by the intake box. The mask leaves the mouth and eyes accessible.

### SUMMARY OF THE INVENTION

The present invention relates to a method of making extended wear contoured facial masks.

It is an object of this invention to provide a method of making a customized, contoured facial mask face surface portion constructed and configured to cover and to contact a corresponding contoured surface area covering substantial surfaces of a human face.

A further object of this invention is to provide methods of making the customized facial mask using three-dimensional (3D) printing methods and materials.

Accordingly, the present invention is defined by the features of claim 1. Further embodiments are defined by the features of claims 2-10.

These and other aspects of the present invention will become apparent to those skilled in the art after a reading of the following description of the preferred embodiment when considered with the drawings, as they support the claimed invention.

### BRIEF DESCRIPTION OF THE DRAWINGS

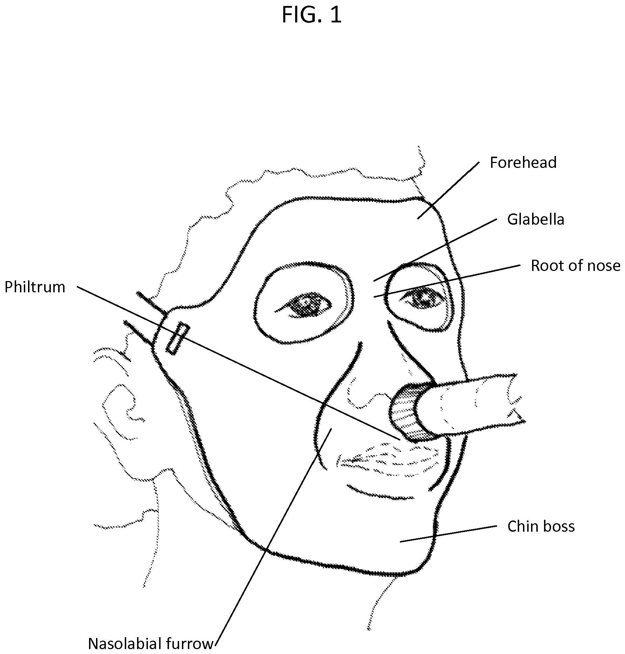

FIG. 1 is a schematic diagram of a perspective view of one example of mask made according to the method of the present invention.

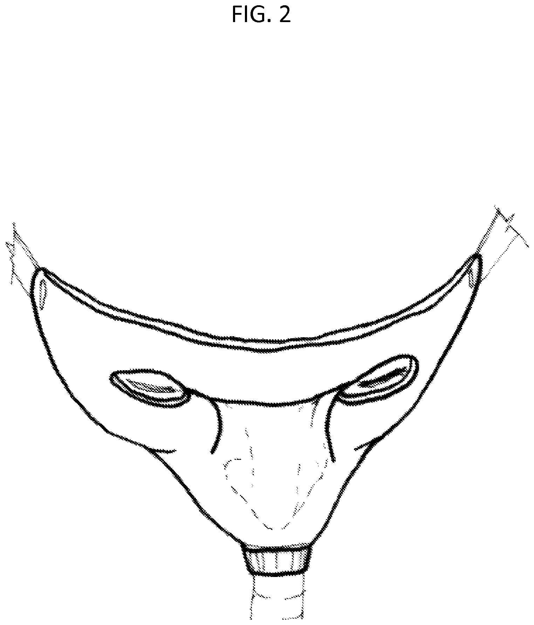

FIG. 2 is a schematic diagram of a top view of one example of mask made according to the method of the present invention.

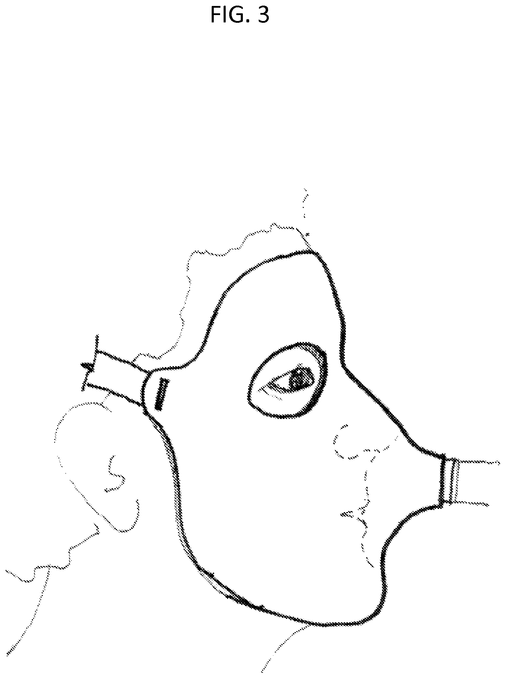

FIG. 3 is a schematic diagram of a side view of one example of mask made according to the method of the present invention.

### DETAILED DESCRIPTION

Referring now to the drawings in general, the illustrations are for the purpose of describing one example of mask made according to the method of the present invention.

By way of further background, the present invention provides a method of making a three-dimensional (3D) printing developed custom face mask component of a sleep apnea treatment device, which addresses the need for customizable masks connected to the machine. The mask used for CPAP devices is an important part of the device, as masks which are not adjusted correctly can result in irritation, bloating, dry mouth and nose, and other problems. The current invention described utilizes 3D printing technology to create customizable masks for sleep apnea patients. Sleep apnea is a disorder, characterized by abnormal breathing during sleep. The abnormality can range from shallow breathing to pauses in breathing while sleeping. If left undiagnosed, other medical conditions may ensue. The disorder is diagnosable by sleep studies conducted in a monitored facility. Sleep apnea symptoms include snoring while sleeping, fatigue during the day and sleepiness during the daytime hours. The solutions for sleep apnea are: surgery, lifestyle changes, mouth pieces, and breathing devices. Breathing devices are the most common form of treatment for sleep apnea patients. The device is comprised of a mask that fits over the nose and mouth or just the nose. The mask is then connected to a machine that blows small amounts of air into the airway to ensure the airway remains open while sleeping. The machine is oftentimes referred to as a Continuous Positive Airway Pressure (CPAP) or Automatic Positive Airway Pressure (APAP).

The method of the present invention provides a customized sleep apnea mask formed utilizing 3D printing technologies for conforming to the unique facial features of the user. The examples of mask obtained according to the method of the present invention are customizable for each user to provide matching contours of the human face for increased comfort when the mask is worn.

In one embodiment of the present invention a 3D model of the anatomically customized mask is created using multidimensional data from an individual's face. The data may be acquired through the use of 3D scanners, multiple image or video cameras and digital reconstruction software, dot or line scans from laser imaging, pattern laser photography, stereo photography, or any number of 3D modeling technologies. Once digitization of the face's 3D surface occurs, an operator may further customize the mask to provide enhanced functionally and aesthetics. For example, and not by way of limitation, the operator may use a computer aided design (CAD) or modeling software to define the boundaries of the mask, create modifications such as strap attachment slit or airway passage, or allow for modular connections such as for a breathing tube.

Since 3D printing involves printing in layers, any digitized model must be mathematically translated into cross-sections or "slices" of the desired print-out. Any intermediate or finalized 3D digital model may be read by the 3D printer for creating the cross-sections or "slices." Furthermore, 3D modeling data can be stored in a database for future use. Such a database, housed in a non-transitory medium such as the memory of a computer, can contain modeling data for individual face contours, modifications to these contours, templates for enhanced functionality or aesthetics, or 3D models of objects to be incorporated into or on to a facial mask. The database of modeling data can be used to reproduce previously printed 3D masks or can be used to adapt such data to a future use, including inventory management, record-keeping, or branding. The database may be accessible through a web interface, providing access to operators, customers, or third parties, with granted access to the database capable of being limited.

A facial mask made according to the method of the present invention is provided for addressing sleep apnea in a user, comprising a customized, contoured facial mask face surface portion constructed and configured to cover and to contact a corresponding contoured surface area of a human face, further including strap attachments and at least one strap for securing the mask portion to the face, wherein the at least one strap is sized to extend around the user's head and for applying a pressure distributed across the contoured facial mask portion, wherein the pressure is distributed substantially uniformly across the contoured facial mask portion that contacts the user's face. Advantageously, the substantial coverage of the face of the user by the mask, preferably at least 50% of the surface area, more preferably at least about 80% of the surface area, provides for reduced pressure points on higher contour areas of the face, and therefore provides for increased comfort to the user. The size of the mask provides that it extends slightly under the chin of the user, wherein a clearance of between about 3.175 mm (1/8 inches) to approximately 6.35 mm (1/4 inches) is provided.

For increased oxygen intake and to facilitate breathing of the user to address issues of sleep apnea, the mask further includes an airway passage positioned in the nose region of the mask, and further including a breathing tube connected to the mask at the airway passage, which extends outwardly from a nasal area of the contoured facial mask face surface portion. In one embodiment, the airway passage and the breathing tube connector is molded into the facial mask. In another embodiment, the breathing tube is removably attachable to the mask, i.e., it is connectable and disconnectable, by a connection region that is matingly connectable. By way of example and not limitation, the connection region includes a threaded zone for rotational connection of the breathing tube with the airway passage.

Importantly, to ensure customized fit of the face mask, the customized, contoured facial mask face surface portion, is constructed and configured to cover and to contact a corresponding contoured surface area of a human face, is unitarily and integrally formed by 3-D printing, and is formed of a synthetic material or plastic. Preferably, a soft plastic layer or a rubber layer is provided on the surface that contacts the face of the user, i.e., that contacts and "mates" with the contours of the user's face, for additional comfort increase. So then both the coverage of the mask over the face of the user and the soft underside layer each separately and in combination provide maximum pressure distribution over the face surface of the user. Thus, the face mask may be formed of a stratified, multilayer structure, wherein the underside layer that contacts the user's face is a softer material than the outer layer, although the layers may be formed integrally together or bonded together.

U.S. Patent Nos. 5869170, 7565633, 7845352, 7963284, 8147910 and 8175734, U.S. Pat. Pub. No. 2012/0224755 and European Patent Nos. 2486547 and 2482248 describe details of 3D printing, materials used for 3D printing, and customizable masks made of plastic or gel materials including but not limited to plastic, living cells, leather, nylon, metal, and thermoplastics.

U.S. Pat. App. No. 2012/0305003 for "Rapid Production Of Customized Masks" by inventor Phillip E. Mark, filed October 21, 2009, describes a system designed for the rapid preparation of anatomically customized mask employing data from a patient. The data may take the form of a multidimensional image of a target area of a patient's face obtained by optical 3 dimensional imaging, or a dot or line scan form laser imaging, pattern laser photography or stereo photography. Also disclosed is a mask that is made of a thin layer, so it is lightweight and closely hugs the targeted region upon which it rests (e.g. the nasal region).

The present invention differs from the prior art, including U.S. Pat. Pub. No. 2012/0305003 in that it is designed to minimize the cosmetic changes to the face of the user upon continual use of the mask. The present invention does this by providing a much larger contact area than the prior art. The contact area includes the large surfaces areas of the face, including the forehead, the brow ridges (supraorbital ridge) and cheeks. The contact area may also include the jaw region.

By extending the mask onto these surfaces, the present invention also allows for the mask to be constructed so that it does not touch the more sensitive parts of the user's face, including the nose and the chin, especially the chin boss. The mask is designed and constructed to not touch the nose and the chin and chin boss such that the user can move the chin while the mask is on. This mobility is found to reduce the irritation of wearing a mask. As previously described, a gap is left between the mask and the chin to permit this movement.

In contrast, the prior art does not extend across the forehead and lower chin, but rather is in contact with and supported by the glabella, root of the nose, and areas directly below the nose such as the philtrum and nasolabial furrow, and the chin.

Thus the prior art teaches both the more sensitive parts of the face supporting the mask, a smaller area of the face supporting the mask and the chin boss supporting the mask. The present invention advantageously teaches a larger support area, less-sensitive parts of the face as support areas, and also provides for chin mobility by not contacting the chin.

Certain modifications and improvements will occur to those skilled in the art upon a reading of the foregoing description. By way of example, the customized facial contour mask made according to the method of the present invention may be adapted for use with cosmetic treatments and/or medical treatments, in particular for time-release or extended-release of beneficial chemicals, vitamins, minerals, and/or topical applications to the face, especially to substantially the entire face surface (excluding the eyes, mouth, and nasal breathing passages). In other embodiments of the present invention, the customized facial contour mask includes the cosmetic and/or medical treatments are applied as a gel, liquid, powder, and/or liner within the inner customized contoured surface of the mask. Additionally or alternatively, the cosmetic and/or medical treatments are integrated within the inner customized contoured surface of the mask.

In alternative embodiments, they may be adapted for use for pilots or firemen, or for the military, for extended-wear oxygen masks or gas masks that provide increased comfort, wherein the masks are customized to the user's facial contours. In other embodiments of the present invention, the customized facial contour mask is adapted to include at least one protective layer. In one example, the protective layer is selected from materials that provide protective qualities including heat-resistant, cut-resistant, impact-resistant, ballistic-resistant, chemical-resistant, and combinations thereof. The protective layer is laminated to and/or affixed to the outer surface of the mask and does not affect the internal customized facial contour mask or its comfort features.

The above-mentioned examples are provided to serve the purpose of clarifying the aspects of the invention and it will be apparent to one skilled in the art that they do not serve to limit the scope of the invention. All modifications and improvements have been deleted herein for the sake of conciseness and readability but are properly within the scope of the present invention.

## Claims

1. A method for making a face mask comprising the steps of:
creating a three-dimensional (3-D) scan having image data of a face of an individual user;
using 3-D printing; and
creating a customized sleep apnea mask for conforming to unique facial features and contours of the face of the individual user; wherein the customized sleep apnea mask includes a contact portion for contacting the face of the individual user;
wherein the contact portion is formed based on the 3D scan having image data of the face of the individual user, and the contact portion is adapted to conform to the unique facial features of the individual user and to substantially contact a forehead of the individual user during use; **characterized in that** the customized sleep apnea mask is sized to extend slightly below a chin of the individual user during use, wherein a clearance of between about 3.175 mm (1/8 inch) and about 6.35 mm (1/4 inch) is provided;
wherein the contact portion is configured to not contact a chin boss of the individual user during use;
wherein the contact portion is configured to not contact a nose of the individual user during use.

2. The method of claim 1, further including the step of saving the scanned image data to a database.

3. The method of claim 2, wherein the database is accessible through a web interface.

4. The method of claim 1, wherein the scanned image data is acquired through use of a 3-D scanner, multiple image or video cameras and digital reconstruction software, dot or line scans from laser imaging, pattern laser photography, or stereo photography.

5. The method of claim 1, wherein the customized sleep apnea mask is configured to not contact and not cover eyes of the individual user during use.

6. The method of claim 1, wherein the contact portion is comprised of a rubber layer.

7. The method of claim 1, wherein the contact portion is configured to contact cheeks of the individual user during use.

8. The method of claim 1, wherein the contact portion is configured to contact supraorbital ridges of the individual user during use.

9. The method of claim 1, wherein the customized sleep apnea mask is sized to cover at least 50% of the surface area of the face of the individual user.

10. The method of claim 1, wherein the customized sleep apnea mask is sized to cover at least 80% of the surface area of the face of the individual user.

## Patentansprüche

1. Ein Verfahren zur Herstellung einer Gesichtsmaske, das die folgenden Schritte beinhaltet:
Erzeugen eines dreidimensionalen Scans (3D-Scans), der Bilddaten eines Gesichts eines einzelnen Benutzers aufweist;
Verwenden von 3D-Drucken; und
Erstellen einer maßgeschneiderten Schlafapnoe-Maske zur Übereinstimmung mit einzigartigen Gesichtsmerkmalen und Konturen des Gesichts des einzelnen Benutzers; wobei die maßgeschneiderte Schlafapnoe-Maske einen Berührungsabschnitt umfasst, um das Gesicht des einzelnen Benutzers zu berühren;
wobei der Berührungsabschnitt basierend auf dem 3D-Scan gebildet ist, der Bilddaten des Gesichts des einzelnen Benutzers aufweist, und der Berührungsabschnitt angepasst ist, um mit den einzigartigen Gesichtsmerkmalen des einzelnen Benutzers übereinzustimmen und eine Stirn des einzelnen Benutzers während des Gebrauchs im Wesentlichen zu berühren; **dadurch gekennzeichnet, dass**
die maßgeschneiderte Schlafapnoe-Maske so bemessen ist, dass sie sich während des Gebrauchs geringfügig unterhalb eines Kinns des einzelnen Benutzers erstreckt, wobei ein Abstand von zwischen etwa 3,175 mm (1/8 Zoll) und etwa 6,35 mm (1/4 Zoll) bereitgestellt ist;
wobei der Berührungsabschnitt so konfiguriert ist, dass er während des Gebrauchs einen Kinnvorsprung des einzelnen Benutzers nicht berührt;
wobei der Berührungsabschnitt so konfiguriert ist, dass er während des Gebrauchs eine Nase des einzelnen Benutzers nicht berührt.

2. Verfahren gemäß Anspruch 1, das ferner den Schritt des Speicherns der gescannten Bilddaten in einer Datenbank umfasst.

3. Verfahren gemäß Anspruch 2, wobei über eine Webschnittstelle auf die Datenbank zugegriffen werden kann.

4. Verfahren gemäß Anspruch 1, wobei die gescannten Bilddaten durch Verwendung eines 3D-Scanners, mehrerer Bild- oder Videokameras und digitaler Rekonstruktionssoftware, Punkt- oder Linienscans von Laserbildgebung, Musterlaserphotografie oder Stereophotographie erfasst werden.

5. Verfahren gemäß Anspruch 1, wobei die maßgeschneiderte Schlafapnoe-Maske so konfiguriert ist, dass sie während des Gebrauchs die Augen des einzelnen Benutzers nicht berührt und nicht bedeckt.

6. Verfahren gemäß Anspruch 1, wobei der Berührungsabschnitt eine Gummischicht beinhaltet.

7. Verfahren gemäß Anspruch 1, wobei der Berührungsabschnitt so konfiguriert ist, dass er während des Gebrauchs Wangen des einzelnen Benutzers berührt.

8. Verfahren gemäß Anspruch 1, wobei der Berührungsabschnitt so konfiguriert ist, dass er während des Gebrauchs supraorbitale Rippen des einzelnen Benutzers berührt.

9. Verfahren gemäß Anspruch 1, wobei die maßgeschneiderte Schlafapnoe-Maske so bemessen ist, dass sie mindestens 50 % der Oberfläche des Gesichts des einzelnen Benutzers bedeckt.

10. Verfahren gemäß Anspruch 1, wobei die maßgeschneiderte Schlafapnoe-Maske so bemessen ist, dass sie mindestens 80 % der Oberfläche des Gesichts des einzelnen Benutzers bedeckt.

## Revendications

1. Une méthode pour fabriquer un masque pour visage comprenant les étapes consistant à :
créer un balayage tridimensionnel (3D) ayant des données d'image d'un visage d'un utilisateur individuel ;
utiliser l'impression 3D ; et
créer un masque sur mesure pour l'apnée du sommeil destiné à épouser des caractéristiques faciales uniques et contours du visage de l'utilisateur individuel ;
dans laquelle le masque sur mesure pour l'apnée du sommeil inclut une portion de contact destinée à être en contact avec le visage de l'utilisateur individuel ;
dans laquelle la portion de contact est formée sur la base du balayage en 3D ayant des données d'image du visage de l'utilisateur individuel, et la portion de contact est conçue pour épouser les caractéristiques faciales uniques de l'utilisateur individuel et pour être substantiellement en contact avec un front de l'utilisateur individuel lors de l'utilisation ;
**caractérisée en ce que**
le masque sur mesure pour l'apnée du sommeil est dimensionné afin de s'étendre légèrement en dessous d'un menton de l'utilisateur individuel lors de l'utilisation, dans laquelle un jeu compris entre environ 3,175 mm (1/8 pouce) et environ 6,35 mm (1/4 pouce) est fourni ;
dans laquelle la portion de contact est configurée pour ne pas être en contact avec une partie saillante du menton de l'utilisateur individuel lors de l'utilisation ;
dans laquelle la portion de contact est configurée pour ne pas être en contact avec un nez de l'utilisateur individuel lors de l'utilisation.

2. La méthode de la revendication 1, incluant en outre l'étape consistant à sauvegarder les données d'image balayée sur une base de données.

3. La méthode de la revendication 2, dans laquelle la base de données est accessible par le biais d'une interface web.

4. La méthode de la revendication 1, dans laquelle les données d'image balayée sont acquises par le biais de l'utilisation d'un dispositif de balayage 3-D, de caméras d'image ou vidéo multiples et d'un logiciel de reconstruction numérique, de balayages par points ou lignes à partir d'une imagerie laser, d'une photographie laser à motif, ou d'une stéréophotographie.

5. La méthode de la revendication 1, dans laquelle le masque sur mesure pour l'apnée du sommeil est configuré pour ne pas être en contact avec et à ne pas recouvrir les yeux de l'utilisateur individuel lors de l'utilisation.

6. La méthode de la revendication 1, dans laquelle la portion de contact est constituée d'une couche en caoutchouc.

7. La méthode de la revendication 1, dans laquelle la portion de contact est configurée pour être en contact avec les joues de l'utilisateur individuel lors de l'utilisation.

8. La méthode de la revendication 1, dans laquelle la portion de contact est configurée pour être en contact avec des bords supraorbitaires de l'utilisateur individuel lors de l'utilisation.

9. La méthode de la revendication 1, dans laquelle le masque sur mesure pour l'apnée du sommeil est dimensionné afin de recouvrir au moins 50 % de la superficie du visage de l'utilisateur individuel.

10. La méthode de la revendication 1, dans laquelle le masque sur mesure pour l'apnée du sommeil est dimensionné afin de recouvrir au moins 80 % de la superficie du visage de l'utilisateur individuel.
